# Europäisches Patentamt
# European Patent Office
# Office européen des brevets

(19)

(11) Veröffentlichungsnummer: **0 103 794**
**B1**

(12) # EUROPÄISCHE PATENTSCHRIFT

(45) Veröffentlichungstag der Patentschrift:
**16.03.88**

(51) Int. Cl.⁴: **G 21 K 1/04**

(21) Anmeldenummer: **83108639.2**

(22) Anmeldetag: **01.09.83**

(54) Strahlenblende für ein Röntgenuntersuchungsgerät.

(30) Priorität: **16.09.82 DE 3234410**

(43) Veröffentlichungstag der Anmeldung:
**28.03.84 Patentblatt 84/13**

(45) Bekanntmachung des Hinweises auf die Patenterteilung:
**16.03.88 Patentblatt 88/11**

(84) Benannte Vertragsstaaten:
**DE FR GB IT**

(56) Entgegenhaltungen:
**DE-A-1 037 035**
**DE-A-2 053 069**
**FR-A-522 956**

(73) Patentinhaber: **Siemens Aktiengesellschaft Berlin und München, Wittelsbacherplatz 2, D-8000 München 2 (DE)**

(72) Erfinder: **Holzermer, Günter, Ebrardstrasse 106, D-8520 Erlangen (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1988

EP 0 103 794 B1

**Beschreibung**

Die Erfindung bezieht sich auf eine Strahlenblende für ein Röntgenuntersuchungsgerät mit mehreren, paarweise gegeneinander verstellbaren, ein Strahlenbündel rechteckigen Querschnittes und unterschiedlicher Größe einblendenden Blendenplatten mit zusätzlichen, in die vier Eckbereiche des Strahlenbündels schwenkbaren Blendenplatten zur Einblendung eines annähernd kreisförmigen Strahlenbündels veränderlichen Durchmessers sowie mit einem Stelltrieb für die zuzätzlichen schwenkbaren Blendenplatten.

Die US-PS 26 75 486 offenbart eine Strahlenblende für Röntgenstrahlen, bei der vier Blendenplatten in einer senkrecht zum Zentralstrahl gelegenen Ebene gegeneinander verschiebbar gelagert sind. Mit dieser strahlenblende lassen sich nur rechteckige Strahlenbündel unterschiedlicher Größe einblenden. Bei der Verwendung von Röntgenbildverstärkern - die bekanntlich einen runden Eingangsleuchtschirm haben - erhält man bei voller Ausleuchtung des Feldes des Eingangsleuchtschirmes hinter solchen Strahlenblenden eine 30 %-ige Überstrahlung an den Ecken des rechteckigen Strahlenbündels. Diese Überstrahlung verringert den Kontrast der Röntgenaufnahme und erhöht die Strahlenbelastung des Patienten.

Es ist durch die DE-AS 10 37 035 auch eine für die Strahlentherapie entwickelte Strahlenblende bekannt, bei der die vier in einer Ebene verschiebbar gelagerten Blendenplatten, eine jede für sich, schräg unterteilt sind. Die beiden annähernd dreieckigen Hälften einer jeden Blendenplatte können bei dieser Strahlenblende mit einer Spindel gegeneinander verschoben werden. Diese Strahlenblende, die eine Einblendung von viereckigen bis maximal achteckigen Feldern erlaubt, ist sowohl in ihrer Herstellung als auch in ihrer Bedienung, bei der fünf Drehgriffe unabhängig voneinander zu verstellen sind, recht aufwendig.

Schließlich ist durch die DE-PS 20 53 089 eine Strahlenblende für Röntgenuntersuchungsgeräte bekanntgeworden, bei der zusätzlich zu den Blendenplatten, die eine rechteckige Einblendung des Strahlenbündels erlauben, in einer weiteren Ebene eine Reihe von dreieckigen, mit ihren Kanten aneinandergleitende Blendenplatten längs einer gekrümmten Führungskurve um die Symmetrieachse der Strahlenblende verstellbar sind. Mit einer solchen Konstruktion lassen sich je nach Anzahl der verwendeten dreieckigen Blendenplatten mehr oder weniger an die Kreisform angenäherte Querschnitte einblenden. Dem Vorzug, dieser Strahlenblende rechteckige wie auch annähernd kreisförmige Querschnitte einblenden zu können, steht die Eigenschaft gegenüber, daß bei der Verstellung, wegen der vielen spaltfrei aneinandergleitenden Kanten, eine beachtliche Reibung überwunden werden muß. Letztere läßt diese Strahlenblende für die Fernsteuerung weniger geeignet erscheinen. Darüber hinaus muß diese Strahlenblende, um ein Verklemmen der dreieckigen Blendenplatten zu vermeiden, regelmäßig gewartet werden.

Eine Strahlenblende, die die Einblendung eines rechteckigen Strahlenbündels erlaubt, wird durch die Fa. Philips vertrieben. Bei dieser Strahlenblende läßt sich die Überstrahlung eines kreisförmigen Eingangsleuchtschirmes dadurch verringern, daß im Bedarfsfall zusätzliche Blendenplatten in mindestens zwei verschiedenen Ebenen in den Strahlenkegel eingeschwenkt werden. Es ist eine Eigenart dieser Strahlenblende, daß die zusätzlichen Blendenplatten in mindestens zwei verschiedenen Ebenen angeordnet werden müssen, damit sie sich bei kleinen Feldern nicht gegenseitig behindern. Auch ist die verbleibende Überstrahlung eines runden Eingangsleuchtschirmes vom Durchmesser des eingeblendeten Strahlenbündels abhängig.

Der Erfindung liegt die Aufgabe zugrunde, eine Strahlenblende für Röntgenuntersuchungsgeräte zu schaffen, mit der sich sowohl rechteckige wie auch annähernd kreisförmige Strahlenfelder einblenden lassen. Eine verbleibende Überstrahlung eines kreisförmigen Eingangsleuchtschirmes soll möglichst klein und für alle Durchmesser gleich groß sein. Die Strahlenblende soll sich zur Fernsteuerung eignen und daher möglichst leichtgängig sein. Darüber hinaus soll die Strahlenblende in Strahlenrichtung möglichst wenig Platz beanspruchen, möglichst wenig Gewicht haben und wartungsfrei sein.

Bei einer Strahlenblende der eingangs genannten Art haben daher erfindungsgemäß die zusätzlichen Blendenplatten einen im wesentlichen dreieckigen Grundriß, sind über ein Parallelogrammgestänge aus je einer seitlich des maximal einblendbaren Querschnittes des Strahlenbündel nahe dessen Ecken gelegenen Warteposition mit einer dem Strahlenbündels zugewandten, senkrecht zur Winkelhalbierenden der jeweiligen Ecke ausgerichteten Kanten annähernd radial auf die Symmetrieachse der Strahlenblende zu schwenkbar. Dies bringt den Vorteil mit sich, daß die für die kreisförmige Ausblendung des Strahlenfeldes erforderlichen zusätzlichen Blendenplatten wegen ihres dreieckigen Grundrisses und der Schwenkung über ein Parallelogrammgestänge sich auch bei der Ausblendung sehr kleiner Durchmesser gegenseitig nicht behindern. So können sie in ein und derselben Ebene angeordnet werden. Schließlich ist die Überstrahlung eines kreisförmigen Eingangsleuchtschirmes wegen der Schwenkung der zusätzlichen Blendenplatten über ein Parallelogrammgestänge und ihrer geraden, den Strahlenkegel begrenzenden Kante unabhängig vom Durchmesser des Eingangsleuchtschirmes.

Der Einsatzbereich der Strahlenblende läßt sich wesentlich erweitern, wenn in besonders vorteilhafter Weiterbildung der Erfindung alle zusätzlichen Blendenplatten längs Bahnen zur Symmetrieachse der Strahlenblende hin verschiebbar sind, auf der sie die Symmetrieachse aus der Sicht der Symmetrieachse mit der jeweils gleichen Ecke erreichen. Dies hat zur Folge, daß sich die zusätzlichen Blendenplatten bei Anordnung in einer einzigen Ebene auch bei starker Einblendung des Strahlenfeldes nicht gegenseitig stören. Somit sind beliebig kleine, annähernd kreisförmige Strahlenfelder einblendbar.

Weitere Einzelheiten der Erfindung werden anhand eines in den Figuren dargestellten Ausführungsbeispieles erläutert. Es zeigen:

Fig. 1 eine vereinfachte Darstellung der teilweise aufgebrochenen Strahlenblende in Seitenansicht, und

Fig. 2 einen Schnitt längs der Linie II-II der Figur 1.

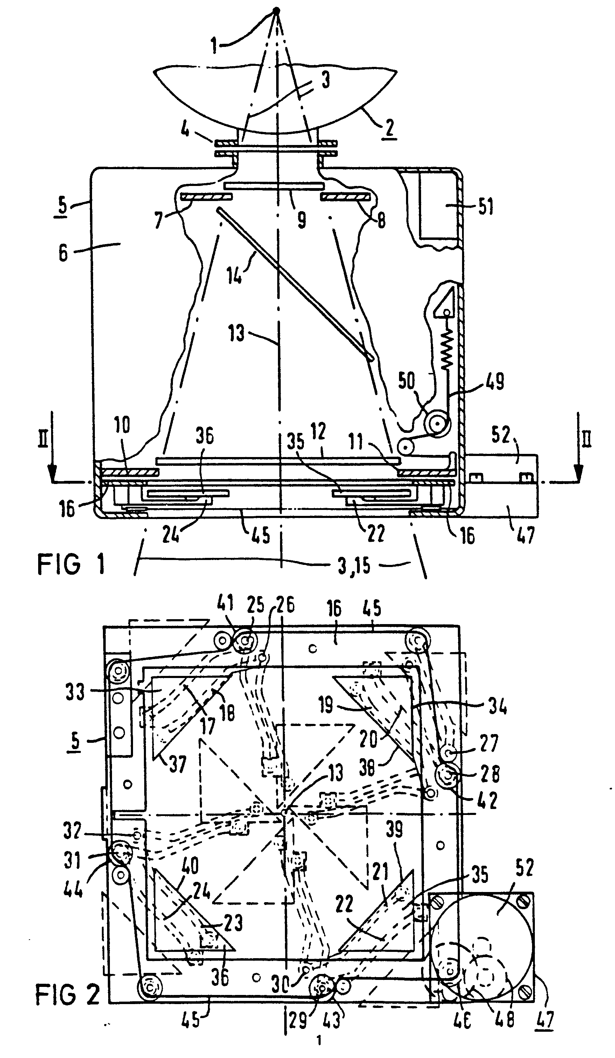

Wie die Figur 1 zeigt, wird der vom angedeuteten Fokus 1 einer Röntgenröhre 2 ausgehende Strahlenbündel 3, soweit es durch den Strahlenaustrittsflansch 4 der Röntgenröhre ins Innere der Strahlenblende 5 gelangt, sowohl von den im aufgebrochenen Gehäuse 6 der Strahlenblende erkennbaren fokusnahen Blendenplatten 7, 8, 9 wie auch von den fokusfernen Blendenplatten 10, 11, 12 (jeweils nur drei Blendenplatten sichtbar) von je vier Seiten rechteckig eingeblendet. Hierzu sind zweimal zwei Paare Blendenplatten senkrecht zur Symmetrieachse 13 der Strahlenblende 5 verstellbar. Die die gleiche Seite des Strahlenbündels 3 begrenzenden, übereinanderstehenden fokusnahen und fokusfernen Blendenplatten sind in hier nicht weiter dargestellter Weise so miteinander gekuppelt, daß sie das gleiche Strahlenbündel begrenzen. Zwischen den fokusnahen und fokusfernen Blendenplatten ist im Gehäuse 6 der Strahlenblende 5 im Bereich des Strahlenbündels 3 ein schräg zur Symmetrieachse 13 der Strahlenblende gestellter Spiegel 14 angeordnet. Eine Lichtquelle (nicht dargestellt) ist im Gehäuse 6 der Strahlenblende so zum Spiegel ausgerichtet, daß der vom Spiegel 14 reflektierte Lichtkegel 15 durch die fokusfernen Blendenplatten 10, 11, 12 deckungsgleich mit dem Röntgenstrahlenbündel 3 eingeblendet wird.

Das Gehäuse 6 der Strahlenblende 5 trägt in einer in Strahlenrichtung unmittelbar hinter den fokusnahen Blendenplatten 10, 11, 12 gelegenen Ebene einen inneren Rahmen 16, an dem, wie die Figur 2 zeigt, acht Auslegerstangen 17, 18, 19, 20, 21, 22, 23, 24 paarweise um parallel zur Symmetrieachse 13 der Strahlenblende ausgerichtete Achsen 25, 26, 27, 28, 29, 30, 31, 32 schwenkbar gelagert sind. An den freien Enden je eines Paares von Auslegerstangen ist eine zusätzliche dreieckige Blendenplatte 33, 34, 35, 36 schwenkbar gelagert. Je zwei Auslegerstangen bilden zusammen mit der zusätzlichen Blendenplatte und dem inneren Rahmen 16 ein Parallelogrammgestänge, mit dem die zusätzliche Blendenplatte in das Strahlenbündel 3 schwenkbar ist. Dabei sind die zusätzlichen Blendenplatten jeweils auf einem Viertelkreis aus einer Ecke des inneren Rahmens 16 der Strahlenblende heraus zur Symmetrieachse 13 der Strahlenblende hin schwenkbar. Die Ausrichtung der zusätzlichen Blendenplatten 33, 34, 35, 36 ist so gewählt, daß sie mit der Kante 37, 38, 39, 40, die senkrecht zur Winkelhalbierenden der jeweiligen Ecke des rechteckigen Strahlenbündels 3 ausgerichtet ist, das Strahlenbündel begrenzt. Die beiden anderen Kanten einer jeden zusätzlichen, dreieckigen Blendenplatte sind dem Eckbereich des inneren Rahmens angepaßt. Infolge der Schwenkbewegung des jeweiligen Parallelogrammgestänges erreichen die vier zusätzlichen Blendenplatten die Symmetrieachse 13 der Strahlenblende 5 mit jeweils ein und derselben Ecke. Im Ausführungsbeispiel der Figur 2 ist das, aus der Sicht der Symmetrieachse 13, die linke Ecke.

Wie die Figuren 1 und 2 erkennen lassen, ist jeweils eine der beiden Auslegerstangen eines Parallelogrammgestänges mit einer Zahnriemenscheibe 41, 42, 43, 44 gekuppelt. Um sämtliche vier Zahnriemenscheiben der Auslegerstange ist ein endloser Zahnriemen 45 gespannt. In einer Ecke des inneren Rahmens 16 ist dieser endlose Zahnriemen über eine Zahnriemenscheibe 46 geführt, die von einem Motor 47 über ein Getriebe 48 antreibbar ist. Eine der fokusfernen, für die rechteckige Ausblendung zuständigen Blendenplatten ist, wie in der Figur 1 angedeutet wird, mit einem federbelasteten Seilzug 49 gekuppelt, über den ein wegabhängiger Analog-Digital-Wandler 50 verstellbar ist. Dessen kodierte Signale liegen an einem Eingang einer Differenzstufe 51 an. Auch der Motor 47 ist mit einem wegabhängigen Analog-Digital-Wandler 52 gekuppelt. Dessen Signale liegen ebenfalls an einem Eingang der Differenzstufe 51 an.

Beim Durchleuchtungs- und Aufnahmebetrieb kann die Strahlenblende 5 wie jede andere Strahlenblende benutzt werden. Die Öffnung des Strahlenbündels ist in an sich bekannter Weise durch synchrones Verstellen der in Strahlenrichtung hintereinander angeordneten fokusnahen und fokusfernen Blendenplatten 7, 8, 9, 10, 11, 12 veränderbar. Dabei blenden die fokusnahen und fokusfernen Blendenplatten ein rechteckiges Strahlenfeld unterschiedlicher Länge und Breite aus. Die zusätzlichen Blendenplatten 33, 34, 35, 36 befinden sich dabei in der in der Figur 2 gestrichelt angedeuteten Warteposition, bei der sie, in der Darstellung der Figur 2, unter den inneren Rahmen 16 geschwenkt sind. Sie stehen in dieser Position nur wenige Millimeter über die inneren Ecken des inneren Rahmens 16 vor, ragen aber nicht in den durch die fokusnahen und fokusfernen Blendenplatten maximal ausblendbaren Strahlenbündel 3. Die Weite des rechteckigen ausgeblendeten Strahlenbündels kann also in Breite und Höhe nach Belieben an die Abmessungen des Untersuchungsbereiches bzw. an das gewählte Kassetten- oder Filmformat angepaßt werden.

Soll mit einem Röntgenbildverstärker gearbeitet werden, so wird auch in diesem Fall die Öffnung des Strahlenkegels durch Verstellen der fokusnahen und fokusfernen Blendenplatten 7, 8, 9, 10, 11, 12 auf den Durchmesser des Eingangsleuchtschirmes des Röntgenbildverstärkers (nicht dargestellt) eingeblendet. In diesem Fall würde jedoch das so eingestellte quadratische Strahlenfeld im Bereich seiner vier Ecken den runden Eingangsleuchtschirm des Röntgenbildverstärkers überstrahlen. Um diese Überstrahlung zu verringern, kann der Motor 47 beispielsweise über ein bei der Umschaltung auf Bildverstärkerbetrieb ansprechendes Relais (nicht dargestellt) eingeschaltet werden. Über den Motor 47 wird der Zahnriemen 45 und über diesen werden die Zahnriemenscheiben 41, 42, 43, 44 der Auslegerstangen so weit gedreht, bis die vom wegabhängigen Analog-Digital-Wandler 52 des Motors 47 erzeugten, an die Differenzstufe 51 anliegenden Signale gleich jenen sind, die vom wegabhängigen Analog-Digital-Wandler 50 der fokusfernen Blendenplatte 11 an der Differenzstufe 51 anliegen. Das heißt, bis die den Strahlenkegel 3 begrenzenden Kanten 37, 38, 39, 40 der einander gegenüberliegenden, zusätzlichen Blendenplatten 33, 34, 35, 36 einen gleich weiten Strahlenbündel 3 begrenzen wie die übrigen Blendenplatten 7, 8, 9, 10, 11, 12. Der Parallaxenunterschied, der sich aus dem unterschiedlichen Fokusabstand der zusätzlichen Blendenplatten 33, 34, 35, 36 und der mit dem Seilzug gekuppelten Blendenplatte 11 ergibt, ist durch das Übersetzungsverhältnis im Stelltrieb zu berücksichtigen.

Es wäre aber auch möglich, die zusätzlichen Blendenplatten 33, 34, 35, 36 mit Hilfe des über den Spiegel 14 auf das Untersuchungsobjekt geworfenen Lichtbündels 15 manuell oder mit Hilfe eines manuell einschaltbaren Motors 47 zu verstellen. Anstelle eines Motors mit einem wegabhängigen Analog-Digital-Wandler kann such ein Schrittmotor und anstelle der Differenzstufe 51 ein Impulszähler verwendet werden, der mit jedem Schritt des Schrittmotors um eine Einheit zurückgezählt wird und von dem der Motor bei der Zählstellung Null abgeschaltet wird.

**Patentansprüche**

1. Strahlenblende (5) für ein Röntgenuntersuchungsgerät mit mehreren, paarweise gegeneinander verstellbaren, ein Strahlenbündel rechteckigen Querschnittes und unterschiedlicher Größe einblendenden Blendenplatten (7, 8, 9, 10, 11, 12) mit zusätzlichen, in die vier Eckbereiche des Strahlenbündels schwenkbaren Blendenplatten (33, 34, 35, 36) zur Einblendung eines annähernd kreisförmigen Strahlenbündels veränderlichen Durchmessers sowie mit einem Stelltrieb für die zuzätzlichen schwenkbaren Blendenplatten (49, 50), dadurch gekennzeichnet, daß die zusätzlichen schwenkbaren Blendenplatten (33, 34, 35, 36) einen im wesentlichen dreieckigen Grundriß haben und über ein Parallelogrammgestänge (16 bis 36) aus je einer, seitlich des maximal ausblendbaren Querschnittes des Strahlenbündels nahe dessen Ecken gelegenen Warteposition mit einer dem Strahlenbündel (3) zugewandten, senkrecht zur Winkelhalbierenden der jeweiligen Ecke ausgerichteten Kante (37 bis 40) annähernd radial auf die Symmetrieachse (13) der Strahlenblende (5) zu schwenkbar sind.

2. Strahlenblende nach Anspruch 1, dadurch gekennzeichnet, daß alle zusätzlichen schwenkbaren Blendenplatten (33, 34, 35, 36) längs Bahnen zur Symmetrieachse der Strahlenblende (5) hin verschiebbar sind, auf der sie die Symmetrieachse (13) aus der Sicht der Symmetrieachse mit der jeweils gleichen Ecke erreichen.

3. Strahlenblende nach Anspruch 1, dadurch gekennzeichnet, daß alle Parallelogrammgestänge (16 bis 36) über einen gemeinsamen Stelltrieb (46, 47, 48) synchron betätigbar sind.

4. Strahlenblende nach Anspruch 3, dadurch gekennzeichnet , daß mindestens eine Auslegerstange (17, 20, 22, 24) eines jeden Parallelogrammgestänges mit einer Zahnriemenscheibe (41, 42, 43, 44) gekuppelt ist und alle Zahnriemenscheiben von ein und demselben Zahnriemen (45) umschlungen sind.

5. Strahlenblende nach Anspruch 4, dadurch gekennzeichnet, daß der Zahnriemen (45) über einen Motor (47) verstellbar ist.

6. Strahlenblende nach Anspruch 5, dadurch gekennzeichnet, daß mindestens ein Paar der das Strahlenbündel (3) rechteckig einblendenden Blendenplatten (7 bis 12) mit einem digitalen Soll-Wegaufnehmer (50) verbunden ist, der Wegaufnehmer an einem Speicher angeschlossen ist und der Motor über den Speicher steuerbar ist.

7. Strahlenblende nach Anspruch 5, dadurch gekennzeichnet, daß der Motor (47) einen Ist-Wegaufnehmer (52) verstellt, der den Motor über ein Vergleichsglied (51), an dem der Soll-Wegaufnehmer (50) angeschlossen ist, steuert.

8. Strahlenblende nach Anspruch 5, dadurch gekennzeichnet, daß der Motor (47) ein Schrittmotor ist.

**Claims**

1. Ray diaphragm (5) for an X-ray examination apparatus with several diaphragm plates (7, 8, 9, 10, 11, 12), adjustable relative to each other in pairs, adjusting a bundle of rays of a rectangular cross-section and of varied size, with additional diaphragm plates (33, 34, 35, 36) able to swivel in the four corner regions of the bundle of rays for adjusting an approximately circular bundle of rays of variable diameter as well as with an adjusting drive (49, 50) for the additional diaphragm plates able to swivel characterised in that
the additional diaphragm plates (33, 34, 35, 36) able to swivel have an essentially triangular outline and, by way of a parallelogram bar (16 to 36), are able to swivel approximately radially towards the axis of symmetry (13) of the ray diaphragm (5), out of a respective holding position, lying to the side of the maximum cross-section of the bundle of rays able to be adjusted, near the corner thereof, with an edge (37 to 40) facing the bundle of rays (3), aligned perpendicularly to the angle bisector of the respective corner.

2. Ray diaphragm according to claim 1, characterised in that
all additional diaphragm plates (33, 34, 35, 36), able to swivel, are displaceable along paths towards the axis of symmetry of the ray diaphragm (5) on which they reach the axis of symmetry (13) along the line of sight of the axis of symmetry with, in each case, the same corner.

3. Ray diaphragm according to claim 1, characterised in that
all parallelogram bars (16 to 36) are able to be actuated synchronously by way of a common adjusting drive (46, 47, 48).

4. Ray diaphragm according to claim 3, characterised in that
at least one cantilever rod (17, 20, 22, 24) of each parallelogram bar is coupled with a cogged belt disc (41, 42, 43, 44) and all cogged belt discs are wrapped around by one and the same cogged belt (45).

5. Ray diaphragm according to claim 4, characterised in that
the cogged belt (45) is adjustable by way of a motor (47).

6. Ray diaphragm according to claim 5, characterised in that
at least one pair of the diaphragm plates (7 to 12) adjusting the bundle of rays (3) in a rectangular manner is connected to a digital desired displacement pick-up (50), the displacement pick-up is attached to a store and the motor is controllable by way of the store.

7. Ray diaphragm according to claim 5, characterised in that the motor (47) adjusts an actual displacement pick-up (52), which controls the motor by way of a comparison member (51), to which the desired displacement pick-up (50) is attached.

8. Ray diaphragm according to claim 5, characterised in that the motor (47) is a step motor.

**Revendications**

1. Diaphragme de rayonnement (5) pour un appareil de radiodiagnostic, avec plusieurs plaques de diaphragme (7, 8, 9, 10, 11, 12) qui sont déplaçables par paires entre elles et qui définissent un faisceau de rayonnement de section transversale rectangulaire et de grandeur différente, avec des plaques de diaphragme supplémentaires, (33, 34, 35, 36) agencées avec basculement dans les quatre zones des sommets pour délimiter un cône de rayonnement à peu près de forme circulaire et de diamètre variable, ainsi qu'avec un mécanisme de réglage (49, 50) pour les plaques de diaphragme supplémentaires et basculables, caractérisé par le fait que les plaques de diaphragme supplémentaires et basculables (33, 34, 35, 36) ont, en projection horizontale, sensiblement une forme triangulaire et sont respectivement basculables, à l'aide d'une tringlerie en parallélogramme (16 à 36), à partir d'une position d'attente située latéralement. par rapport au faisceau de rayonnement maximum pouvant être diaphragmé située près des sommets dudit faisceau, à peu près radialement sur l'axe de symétrie (13) du diaphragme de rayonnement (5), avec un bord (37 à 40) voisin du faisceau de rayonnement et perpendiculaire à la bissectrice du sommet correspondant.

2. Diaphragme de rayonnement selon la revendication 1, caractérisé par le fait que toutes les plaques de diaphragme supplémentaires (33, 34, 35, 36) sont susceptibles de se déplacer le long de pistes, vers l'axe de symétrie du diaphragme de rayonnement (5) sur lesquelles elles atteignent l'axe de symétrie (13), et en regardant l'axe de symétrie, avec respectivement le même sommet.

3. Diaphragme de rayonnement selon la revendication 1, caractérisé par le fait que toutes les tringleries en parallélogramme (16 à 36), sont susceptibles d'être commandées de façon synchrone à l'aide d'un mécanisme de réglage commun (46, 47, 48).

4. Diaphragme de rayonnement selon la revendication 3, caractérisé par le fait qu'au moins une tige de commande (17, 20, 22, 24) de chacune des tringleries en parallélogramme, est accouplée à un galet à courroie dentée (41, 42, 43, 44) et que tous les galets à courroie dentée sont entourés par une seule et même courroie dentée (45).

5. Diaphragme de rayonnement selon la revendication 4, caractérisé par le fait que la courroie dentée (45) est déplaçable par un moteur (47).

6. Diaphragme de rayonnement selon la revendication 5, caractérisé par le fait qu'au moins une paire des plaques de diaphragme (7 à 12) qui délimitent le faisceau de rayonnement (3) rectangulaire, est reliée à un capteur de déplacement de consigne numérique, le capteur de déplacement étant relié à une mémoire et le moteur étant susceptible d'être commandé par la mémoire.

7. Diaphragme de rayonnement selon la revendication 5, caractérisé par le fait que le moteur déplace un capteur de déplacement instantané (52), lequel capteur commande, par l'intermédiaire d'un comparateur (51) auquel est relié le capteur de déplacement de consigne.

8. Diaphragme de rayonnement selon la revendication 5, caractérisé par le fait que le moteur est un moteur pas-à-pas.

FIG 1

FIG 2